# EUROPEAN PATENT SPECIFICATION

(11) **EP 3 009 161 B1**
(45) Date of publication and mention of the grant of the patent: **04.03.2020**
(21) Application number: 14004110.4
(22) Date of filing: 05.12.2014
(51) Int. Cl.: A61M 25/00, A61M 25/01, A61M 25/10

(54) **Intravascular microcatheter for delivering active substances**
Intravaskulärer Mikrokatheter zur Abgabe von Wirkstoffen
Microcathéter intravasculaire d'administration de substances actives

(30) Priority: 15.10.2014 PL 40980414
(43) Date of publication of application: 20.04.2016
(73) Proprietor: Innovations for Heart and Vessels Sp. z o.o., 40-171 Katowice (PL)
(72) Inventor: Buszman, Piotr, 40-761 Katowice (PL); Buszman, Pawel, 40-748 Katowice (PL); Milewski, Krzysztof, 40-748 Katowice (PL)
(74) Representative: Lukaszyk, Szymon

(56) References cited:
- WO-A2-2005/049110
- US-A1- 2005 137 579
- US-A1- 2010 174 183

## Description

The subject of the present invention is an intravascular microcatheter for delivering active substances. The invention relates to the field of medical devices for percutaneous insertion into patients' blood vessels, for example, through a radial or femoral artery in order to insert a catheter in place, where lesions are present or where, for other reason, there is a need for a local application of active substances into the interior of the vessel.

In many diseases the systemic drug administration is applied in the first instance, which might cause many side effects, and sometimes does not provide the expected results. In case the lesions occur locally, for example, in specific sections of the blood vessel, it is desirable to use the medications only at the place of destination. In such a situation, it is easier to control the drug delivery and to obtain the desired therapeutic dose and/or the desired concentration of active substance at the location of lesions.

There are known infusion catheters that are provided with pores for delivering liquids, especially active substances, into the interior of the vessels, although these catheters are usually bigger with pores of bigger sizes that are intended for applications other than intravascular, where the active substance flows out under the pressure applied by the operator. The pressure can be uneven and sometimes too rapid, which can cause the uncontrolled movements of the catheter.

From the European Patent Application EP0778036A1 there is known a microcatheter that is porous at its distal part and has the outside diameter of 1 to 0.3 mm. However, the pores of the catheter do not provide any connection between the inside of the catheter and the lumen of the vessel, until the delivered liquid active substance is not provided under the appropriate pressure. For such conditions a flexible structure of the catheter ending is adapted, and it can be expanded at the elevated pressure inside the catheter. The active substance is applied at the pressure of 14 to 69 bar (200 to 1000 PSI), which in a case of the intravascular application might result in the damage to the vessel. The catheter at its distal part might have a variable diameter.

From the United States Patent Application US20030045866A1 there is known a catheter comprising a few longitudinal regions covered with a microporous membrane that becomes permeable after reaching a certain limit in the fluid pressure inside the catheter and as a result the local delivery of therapeutic substance is possible. The drug delivery simultaneously into a few regions is provided.

US 2010/174183 A1 discloses an intravascular catheter for delivering multiple fluids like a contrast agent and saline into the bloodstream, wherein the catheter comprises a proximal end portion, a stem, and a distal end portion with an atraumatic tip. Claim 1 is delimited against this prior art.

The purpose of the present invention is to design an improved microcatheter with a high number of regularly distributed pores of small sizes, which will provide a slow, safe, local delivery of active substances over the entire length of the vessel to cover its walls uniformly. The purpose of the invention is also to improve the microcatheter efficiency by applying an inflatable balloon for closing the lumen of the blood vessel into the region, where the active substance is delivered, in order to stabilize the microcatheter and to prevent the backflow of the active substance and mixing thereof with the inflowing blood.

An intravascular microcatheter for delivering active substances comprising a proximal end portion, a stem, and a distal end portion with an atraumatic tip, according to the present invention is characterised in that it comprises a main channel for delivering active substances centrally positioned and parallel to a lateral guidewire channel arranged at least on the section of the distal end portion of the microcatheter in which there is placed an active substance delivery region with regularly distributed pores in a number of 20 to 200, whereas the atraumatic tip of the microcatheter is devoid of pores and closed.

The outer diameter of the catheter is from 0.5 to 1.5 mm.

In addition, the catheter comprises an atraumatic balloon (**9**) positioned before the region (**4**) at the proximal end portion of the microcatheter.

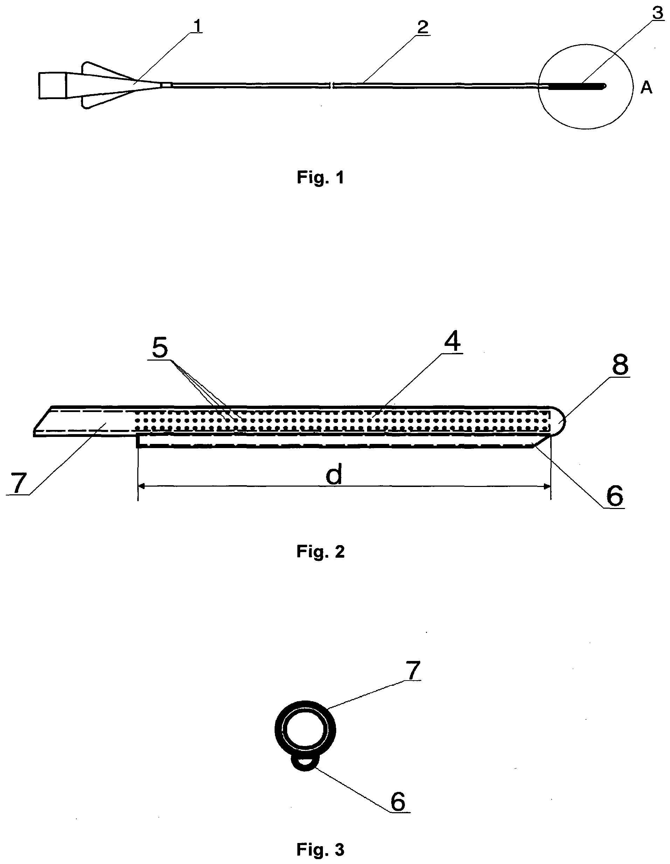

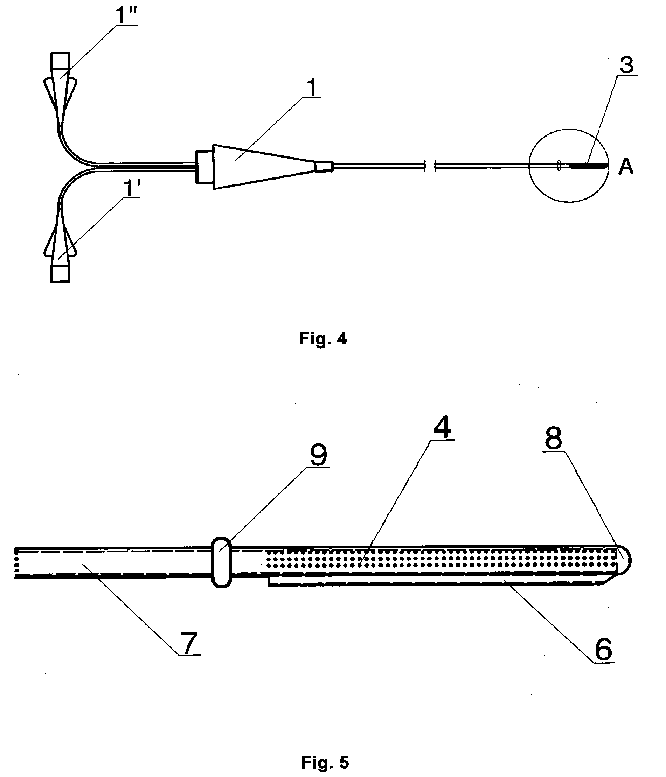

The subject of the present invention in an exemplary embodiment is illustrated in the drawing, where Fig. 1 shows a general view of the microcatheter with its main elements; Fig. 2 shows in magnification the part A of the microcatheter as marked in Fig. 1, i.e. the distal end portion of the microcatheter with the micropores; Fig. 3 shows the cross-section of the distal end portion of the microcatheter as presented in Fig. 2; Fig. 4 shows the general view of the microcatheter in the second exemplary embodiment with a balloon antecedent to the distal end portion of the microcatheter; and Fig. 5 shows in magnification the part A of the microcatheter as marked in Fig. 4, i.e. the distal end portion of the microcatheter with the micropores and the inflated balloon.

The intravascular microcatheter for delivering active substances in an exemplary embodiment comprises the following parts: a proximal end portion **1**, a stem **2**, and a distal end portion **3**, whereas the distal end portion comprises an active substance delivery region **4** with multiple regularly distributed (i.e. with maintaining the fixed, determined distance between them) pores **5.** The pores **5** are located on the lateral surface of the microcatheter in the region **4**, whereas the direction of conducting the pores through the wall of the microcatheter is perpendicular to the main channel **7**, so that the active substance is distributed in a radial direction. The proximal end portion of the microcatheter can be provided with a separate first tip **1'** designed for cooperation with a syringe or another system for inflation and deflation of the balloon and a second tip **1"** that cooperates with a syringe or another system for delivering an active substance solution. Along the microcatheter, there is placed an internal main channel **7**, preferably with a circular cross-section, i.e. a channel for delivering active substances, by which the active substance is delivered (a drug solution or a solution of drug composition). The pores **5** are connected with the main channel **7**, to enable the active substance distribution. Preferably, the distal end portion comprises at least 20 pores, for example, several dozens of pores, also preferably up to 100 or even more than 100 pores, also preferably up to 200 pores. The microcatheter is also provided with a guidewire channel **6** that is parallel to the main channel **7** for delivering active substances and is arranged at least on the section of the distal end portion of the microcatheter. The guidewire channel **6** has smoothly shaped inlet and outlet openings. The guidewire channel **6** has a smaller diameter than the main channel **7** and is located on the lateral wall of the main channel, i.e. the guidewire channel occupies only a small area of the lateral wall of the catheter. This guidewire channel can be led along the whole region with pores **5** for delivering active substances, but can be also shortened and located, for example, only in the terminal part of the region **4** for delivering active substances. The microcatheter is further provided with a closed (made of solid material, devoid of pores), atraumatic, rounded (ovoid) tip **8**, preferably covered with an elastic hydrophilic material to facilitate passing the microcatheter through the winding blood vessel sections. The embodiment as described above is presented in Figs.1, 2 and 3.

In Figs. 4 and 5 the microcatheter is presented in a different variety of implementation, in which it is additionally equipped with an atraumatic inflatable balloon **9** with a circular cross-section. The balloon **9** is located concentrically around the channel for delivering active substances and is designed for the proximal occlusion of the vessel. This allows for more efficient delivery of active substances to the destination place only, prevents the backflow of the active substances which is especially beneficial in case toxic medicaments like cytostatics are applied, and also stabilizes the catheter. The closure of the vessel with the balloon also minimizes the decrease in concentration of the medicament applied locally. The balloon, at the time of conducting the catheter through the blood vessels, is deflated and has the minimum diameter. After filling the balloon and obtaining the occlusion effect, the balloon is easily emptied and returns to its minimum size. For those skilled in the art it would be clear that the microcatheter in its variety with a balloon must have an additional internal channel designed for filling and emptying the balloon, i.e. in the part of the microcatheter that is extending to the place of balloon attachment there are located two channels - a channel for supplying and discharging the liquid to the balloon and a channel leading to the distal end portion which delivers the active substances.

The pores, which are provided in the distal end portion of the microcatheter, have a diameter of 0.1 to 0.5 mm and at least some of these pores are located around the walls of the catheter, and opposed to each other to compensate mutually the pressure of the liquid streams coming out of individual pores. The pores can be arranged diversely, for example, in rows, linearly along the distal end portion or obliquely (or spirally). The pores are made in the wall of the microcatheter in the direction approximately perpendicular to the catheter channel and occupy the region of length **d** - as marked on Figs. 2 - ranging from 5 to 15 cm, for example, 6, 8, 10 cm. The outer diameter of the microcatheter ranges from 0.5 to 1.5 mm, preferably 0.5 to 1.0 mm. The atraumatic distal tip of the catheter does not contain pores, and this allows eliminating the uncontrolled movement of the microcatheter (e.g. the catheter retraction) what is observed if the liquid outflows from the top terminal part of known catheters with a pore/pores on the top of the catheter.

The microcatheter according to the invention can be manufactured using different materials in its various parts. It is possible, in particular, manufacturing of the distal end portion using more flexible and softer materials then the other part of the catheter.

The design of the microcatheter in part where there is the guidewire channel arranged parallel to the channel for delivering active substances may be slightly different then presented in Fig. 3. It is possible to design this part in such way, to obtain a circular cross-section of the catheter, and the two channels - the guidewire channel and the channel for liquid supply - can be adjacent with each other.

The construction of the proximal end portion of the catheter, that is accessible to the operator, such as the ending for connecting the catheter to the pump for delivery of active substances and the pump for balloon inflation, will be apparent to the person skilled in the art. These elements do not constitute the essence of the present solution.

The active substances applied in a liquid form that are delivered by means of the microcatheter according to the invention may be selected from the group consisting of immunosuppressants, cytostatic agents, antiproliferative drugs, anti-inflammatory drugs, antigrowth drugs, antithrombotic drugs, biologic medicaments including tissue adhesive or combinations of these medicaments.

The cytostatic agents may be selected, for example, from the taxanes group (preferably paclitaxel or docetaxel). The immunosuppressants may be selected from the group of rapamycin derivatives, for example, it may be sirolimus, everolimus or zotarolimus.

In comparison to the available and known similar catheters, the microcatheter of the present invention is characterised in that the drug administration is performed over the entire length of the vessel atraumatically-without any contact with the vessel wall by spraying. Due to the use of a "monorail" type of a system, and because of its low profile, it will be possible to administer the drug outside of the occlusion or in place with the lack of blood flow, for example, caused by the phenomenon of "no reflow", where the administered substances does not reach. The presence of the proximal balloon prevents the "leakage" of the drug proximate to the systemic circulation and increases the concentration of active substances in the desired location.

### Reference numerals on the drawings:

- 1: proximal end portion of microcatheter
- 1': proximal end portion of channel for inflating balloon
- 1": proximal end portion of channel for delivering active substances
- 2: stem of microcatheter
- 3: distal end portion of microcatheter
- 4: region of active substance delivery
- 5: pores
- 6: lateral guidewire channel
- 7: main channel for delivering active substances
- 8: tip of microcatheter
- 9: balloon
- d: length of region with pores

## Claims

1. An intravascular microcatheter for delivering active substances comprising a proximal end portion (**1**), a stem (**2**), and a distal end portion (**3**) with an atraumatic tip (**8**), **characterised in that** it comprises a main channel (**7**) for delivering active substances centrally positioned and parallel to a lateral guidewire channel (**6**) arranged at least on the section of the distal end portion (**3**) of the microcatheter in which there is placed an active substance delivery region (**4**) with regularly distributed pores (**5**) in a number of 20 to 200, whereas the atraumatic tip (**8**) of the microcatheter is devoid of pores and closed, and wherein the guidewire channel (**6**) has a smaller diameter than the main channel (**7)** and is located on the lateral wall of the main channel (**7**).

2. The intravascular microcatheter according to claim 1, **characterised in that** the outer diameter of the catheter is from 0.5 to 1.5 mm.

3. The intravascular microcatheter according to claim 1 or 2 **characterised in that** the catheter additionally comprises an atraumatic balloon (**9**) positioned before the active substance delivery region (**4**) at the proximal end portion (**1**) of the microcatheter.

## Patentansprüche

1. Ein intravaskulärer Mikrokatheter zur Verabreichung von Wirkstoffen mit einem proximalen Endteil (1), einem Stamm (2), und einem distalen Endteil (3) mit einer atraumatischen Spitze (8), **dadurch gekennzeichnet, dass** er einen Hauptkanal (7) zur Abgabe von Wirkstoffen umfasst, der zentral und parallel zu einem seitlichen Führungsdrahtkanal (6) mindestens auf dem Abschnitt des distalen Endteils (3) des Mikrokatheters angeordnet, in dem sich ein Wirkstoffabgabebereich (4) befindet, mit gleichmäßig verteilten Poren (5) in einer Anzahl von 20 bis 200, während die atraumatische Spitze (8) des Mikrokatheters porenfrei und geschlossen ist, und wobei der Führungsdrahtkanal (6) einen kleineren Durchmesser als der Hauptkanal (7) hat und sich an der Seitenwand des Hauptkanals (7) befindet.

2. Der intravaskulärer Mikrokatheter nach Anspruch 1, **dadurch gekennzeichnet, dass** der Außendurchmesser des Katheters 0,5 bis 1,5 mm beträgt.

3. Der intravaskulärer Mikrokatheter nach Anspruch 1 oder 2, **dadurch gekennzeichnet, dass** der Katheter zusätzlich einen atraumatischen Ballon (9) umfasst, vor dem Wirkstoffabgabebereich (4) angeordnet, am proximalen Endteil (1) des Mikrokatheters.

## Revendications

1. Un microcathéter intravasculaire pour l'administration de substances actives comprenant une partie d'extrémité proximale (1), une tige (2) et une partie d'extrémité distale (3) avec une pointe atraumatique (8), **caractérisé en ce qu'**il comprend un canal principal (7) pour l'administration de substances actives positionné de manière centrale et parallèle à un canal de fil-guide (6) latéral agencé au moins sur la section de la partie d'extrémité distale (3) du microcathéter dans laquelle est placée une zone d'administration de substance active (4) avec des pores (5) régulièrement répartis en un nombre de 20 à 200, tandis que la pointe atraumatique (8) du microcathéter est dépourvue de pores et fermée, et que le canal de fil-guide (6) a un diamètre plus petit que le canal principal (7) et est situé sur la paroi latérale du canal principal (7).

2. Le microcathéter intravasculaire selon la revendication 1, **caractérisé en ce que** le diamètre extérieur du cathéter est de 0,5 à 1,5 mm.

3. Le microcathéter intravasculaire selon la revendication 1 ou 2, **caractérisé en ce que** le cathéter comprend en outre un ballon atraumatique (9) positionné avant la zone d'administration de substance active (4) à la partie d'extrémité proximale (1) du microcathéter.
